# EUROPEAN PATENT SPECIFICATION

(11) **EP 1 409 053 B1**
(45) Date of publication and mention of the grant of the patent: **03.01.2007**
(21) Application number: 02746250.6
(22) Date of filing: 28.06.2002
(51) Int. Cl.: A61M 15/00

(54) **REMOVING DOSE ELECTRIC CHARGE**
ENTFERNUNG ELEKTRISCHER LADUNG VON EINER DOSIS
ELIMINATION D'UNE CHARGE ELECTRIQUE DANS UNE DOSE

(30) Priority: 13.07.2001 SE 0102522
(43) Date of publication of application: 21.04.2004
(73) Proprietor: Mederio AG, 6052 Hergiswil NW (CH)
(72) Inventor: NILSSON, Thomas, S-647 32 Mariefred (SE)
(74) Representative: Stenborg, Anders Vilhelm
(86) International application number: PCT/SE2002/001284
(87) International publication number: WO 2003/006093

(56) References cited:
- US-A- 5 714 007
- US-A- 6 007 630

## Description

### TECHNICAL FIELD

The present invention relates to a method and a device for quick neutralization of a created electrostatic field formed by a multitude of basically charged particles comprising a medication powder deposited onto a defined target area of a substrate member in a process of forming a pre-metered dose suitable for inhalation.

### BACKGROUND

The dosing of drugs is carried out in a number of different ways in the medical service today. Within health care there is a rapidly growing interest in the possibility of dosing medication drugs as a powder directly to the airways and lungs of a patient by means of an inhaler in order to obtain an effective, quick and user-friendly administration of such substances.

A dry powder inhaler, DPI, represents a device intended for administration of powder into the deep or upper lung airways by oral inhalation. A deep lung deposition is desirable for systemic delivery of medication drugs, but for local treatment of the airways, the objective is local deposition, not deep lung. With deep lung should be understood the peripheral lung and alveoli, where direct transport of active substance to the blood can take place. In order for a particle to reach into the deep lung the aerodynamic particle size should typically be less than 3 µm, and for a local lung delivery typically less than 5 µm. Larger particle sizes will easily stick in the mouth and throat, which underlines the importance of keeping the particle size distribution of the dose within tight limits to ensure that a high percentage of the dose actually is deposited in the deep lung upon inhalation when the objective is systemic delivery of a drug. Furthermore, the inspiration must take place in a calm manner to decrease air speed and thereby reduce deposition in the upper respiratory tracts.

To succeed with systemic delivery of medication powders to the deep lung by inhalation there are some criteria, which have to be fulfilled. It is for instance very important to obtain a high dosing accuracy in each administration to the user. A very high degree of de-agglomeration of the medication powder is also of great importance. This is not possible with dry powder inhalers of today without special arrangements as for example a so-called spacer.

Powders for inhalers have a tendency of agglomerating, in other words to clod or to form smaller or larger lumps, which then have to be deagglomerated. De-agglomeration is defined as breaking up agglomerated powder by introducing electrical, mechanical, or aerodynamic energy. Usually de-agglomeration is performed in at least two stages: stage one is in the process of depositing powder while building up the dose and stage two is in the process of dispersing the powder during the patient's inspiration of air through the DPI.

The term electro-powder refers to a finely divided medication powder presenting controlled electric properties being suitable for administration by means of an inhaler device. Such an electro-powder provides possibilities for a better dosing from equipment using a technique for electric field control such as disclosed in our U.S. Patent No. 6,089,227 as well as our Swedish Patents No. 9802648-7 and 9802649-5, which present excellent inhalation dosing performance. The state of the art also discloses a number of solutions for depositing powder for dosing. The International Application WO 00/22722 presents an electrostatic sensing chuck using area matched electrodes. U.S. Patent No. 6,063,194 discloses a powder deposition apparatus for depositing grains on a substrate using an electrostatic chuck having one or more collection zones and using an optical detection for quantifying the amount of grains deposited. U.S. Patent No. 5,714,007 and U.S. patent No. 6,007,630 disclose an apparatus for electrostatically depositing a medication powder upon predefined regions of a substrate, the substrates being used to fabricate suppositories, inhalants, tablet capsules and the like. In U.S. Patent No. 5,699,649 and U.S. Patent No. 5,960,609 are presented metering and packaging methods and devices for pharmaceuticals and drugs, the methods using electrostatic photo technology to package microgram quantities of fine powders in discrete capsule and tablet form.

A common difficulty encountered when using electrostatic technology and/or electrical fields in combination with electrostatic charging of the powder particles in a deposition process, is to neutralize the created electrostatic field formed by the multitude of deposited particles and the charge of the substrate, if an isolator, as the particles are being deposited on the substrate for forming the dose. If the neutralization of charges is incomplete or takes too long it will affect the forming of the dose negatively in that the charged particles already deposited will present a local repelling electric field, which tends to stop newly attracted particles from settling on the targeted area of the substrate and forces newcomers to settle at the outskirts of the target area. The repelling field grows in strength as more particles are deposited on the target area. Finally, the field is so strong that further deposition is not possible even if the net field strength at some distance from the target area is exerting an attractive force on the charged particles.

In cases where electrostatic chucks are used, regardless of whether the chuck substrate, normally of a dielectric material, is pre-charged in the deposition area or areas to create the necessary local electric field in the target area(s), or a system of electrodes are used to attract the charged particles or if a combination of pre-charging and electrodes are used, it is always difficult to fill the target area with the correct amount of particles, because the repelling field grows stronger with every particle deposited, leading to a spreading out of particles over a larger area than the intended target area. This is also true where the target areas, the deposition areas, constitute beads, which are captured and held by the chuck by for instance electrostatic forces during the deposition of particles onto the beads themselves. It is thus often impossible to form doses of sufficient mass and suitable spatial shape. Often, the chuck principle also requires powders of predetermined or known specific charge (µC/g) in order to predict the mass of particles attracted to the chuck, which by itself presents a big challenge.

Further, prior art technology devices seldom reach a sufficiently high degree of de-agglomeration, and an exact dose with a low relative standard deviation (RSD) between doses is not well controlled. This is partly due to difficulties in controlling the production line parameters during production of the doses, partly to shortcomings in the design of the inhaler device, which make it hard to comply with regulatory demands. The difficulties leave much to be desired when it comes to dose conformity and lung deposition effectiveness of the medication substance. Therefore, there is still a demand for prefabricated high accuracy pre-metered doses to be loaded into an inhaler device, which then will ensure repeated and exact systemic or local pulmonary delivery of doses administered by inhalation.

### SUMMARY

A method and a device are defined for quick neutralization of a created electrostatic field formed by a multitude of basically charged particles comprising a medication powder deposited onto a defined target area of a substrate member in the course of a dose forming process.

A source of charges or a charge generator, not to be confused with the particle generator, is arranged such that the emitted charges, positive or negative or both, are directed towards the target area of the substrate member so that the electric field created by the accumulating charges from a multitude of particles is neutralized by the added charges. Various means, e.g. corona, induction or tribo effect may be used to generate equalizing charges, but in a preferred embodiment, an ion source has been found to be most efficient in achieving neutralization of the dose charge and the substrate. The chosen method or device is selected not to affect the powder substance in any other way but to neutralize the electric charges.

The source is applied so that the dose and the target area are exposed to the emitted charges during the whole or part of the dose forming process. Alternatively, the source may be positioned out of range of the target area such that the substrate member with the target area or even the source itself is repositioned by a servo device when a neutralization of accumulated charge in the dose is needed. For best results, it is necessary to choose a source of suitable strength and provide adequate screening to direct the charges towards the dose or, if the target area is larger than the area where the dose is formed, towards the part of the dose where the deposition is taking place.

Making electric contact with the electrode behind the target area on the substrate member is sometimes difficult because of the physical requirements put on the substrate member by the servo device and control system. Restrictions may exist in terms of acceptable materials, physical implementations etc, which make standard methods of making electric contact difficult or impossible to use. The present invention provides a noncontact electric connection by using yet a further ion source, put behind the substrate member. The ion source ionizes the gas, normally air, between the electrode and the servo device controlling the movements of the substrate member. Then, if a voltage source is arranged feeding the ion source chassis with an appropriate voltage the voltage also will appear as a potential on the electrode, without too much voltage loss. In this manner the electrode is included in the circuit and will work as intended in attracting powder particles to the target area.

A method according to the present invention is set forth by the independent claim 1 and further embodiments of the method are set forth by the dependent claims 2 to 8. A device for removing charges is set forth by the independent claim 9 and further embodiments are defined by the dependent claims 10 to 17.

### BRIEF DESCRIPTION OF THE DRAWINGS

The invention, together with further objects and advantages thereof, may best be understood by referring to the following detailed description taken together with the accompanying drawings, in which:
- FIG. 1: illustrates in principle how the deposition of charged particles onto the target area of the substrate member is made more and more difficult and erratic because of the increasing repelling electric field created by the accumulated charge of the multitude of already deposited particles;
- FIG. 2: illustrates in principle a first embodiment of an iris diaphragm/ shutter comprising one electrode only, showing how charged particles are being transferred from the particle generator to the target area of the substrate member;
- FIG. 3: illustrates the same embodiment as in Figure 2 but with the transfer of particles inhibited by a repelling acting electric field from the electrode of the iris diaphragm/ shutter;
- FIG. 4: illustrates in principle a second embodiment of an iris diaphragm/shutter comprising two electrodes, showing how charged particles are transferred from the particle generator to the target area of the substrate member;
- FIG. 5: illustrates a typical embodiment of an iris diaphragm/shutter comprising two electrodes;
- FIG. 6: illustrates in principle a third embodiment of an iris diaphragm comprising four electrodes, showing how charged particles are transferred from the particle generator to the target area of the substrate member, which may be moved by a servo mechanism during dose forming;
- FIG. 7: illustrates in principle one side of a typical iris diaphragm showing a second electrode;
- FIG. 8: illustrates in principle one side of a typical iris diaphragm showing a first electrode;
- FIG. 9: illustrates in principle an iris diaphragm with two electrodes, a dose being formed onto the target area of the substrate member and two ion sources for removing accumulated charge in the dose being formed;
- FIG. 10: illustrates in principle an iris diaphragm with two electrodes, a dose being formed onto the target area of the substrate member, a servo arrangement for moving the substrate member in relation to the iris and an ion source for removing accumulated charge in the dose being formed;
- FIG. 11: illustrates, taken together with Fig. 10, in principle a substrate member in the shape of a revolving cassette with more than one target area, doses being formed onto the target areas and an ion source for removing - one by one - accumulated charges in the doses being formed; and
- FIG. 12: illustrates schematically a substrate member, an iris diaphragm, a dose in forming and an ion source positioned behind the substrate member connecting without physical contact the third voltage source with the third electrode.

### DESCRIPTION OF THE INVENTION

A method and a device involving a source of charges are disclosed for quick neutralization of a created electrostatic field formed by a multitude of charged particles of a medication powder deposited onto a defined target area of a substrate member in the course of a dose forming process. Spatial distribution of particles onto the target area or dose bed is achieved by means of electro-dynamic field technique applied to the distribution and deposition of particles in a dose forming process. The term "electro-dynamic field technique" in the context of this document refers to the effective electric field in four dimensions, space and time, resulting from well controlled - in terms of timing, frequency and amplitude - potentials applied to a number of electrodes placed in suitable positions in the space confined by a dose forming apparatus. The term "quasi-stationary electric field" is in this context used to describe an electric field or fields being controlled by voltage source devices with controlled impedance, all part of a control system, where the applied voltages may be controlled arbitrarily and individually in the low-frequency time-domain.

To facilitate the understanding of where and how voltages are applied all voltages are assumed to be referenced to ground potential throughout this document. Naturally, ground potential may be exchanged for an arbitrary potential when utilizing the invention and it will be apparent to a person skilled in the art that any singular potential or voltage may be referenced to another potential or voltage source, e.g. in order to simplify or improve a control system, without departing from the scope of the invention as defined in the appended claims.

A powder particle generator is provided, which either produces positively and/or negatively charged powder particles by corona-, tribo- or induction-charging. The charged particles are emitted from the generator into a controlled atmosphere, normally air, where they enter an electric field coming from suitably positioned electrodes at suitable potentials supplied by controlled voltages from suitable voltage sources. At least one of the electrodes comprises an electric iris diaphragm/ shutter. The iris diaphragm/ shutter has at least one aperture of suitable size and shape where particles can pass through and it is positioned between the particle generator and the substrate. In a typical embodiment, the iris diaphragm comprises two electrodes with a thin isolating wafer member between them, and a single aperture through the iris diaphragm. The electrodes and the isolating wafer member are typically made as a printed circuit board (PCB) with topside and bottom side. The electrode (topside by definition) closest to the substrate member is typically circular in shape and concentric with the aperture, while the other electrode (bottom side by definition) is closest to the particle generator and may cover the lower side of the PCB completely. In a preferred embodiment, the substrate member is positioned upside down above the particle generator such that the net electrostatic force acting on emitted charged particles is directed upwards counteracting the force of gravity during forming of the dose. In this manner no big or heavy particles can land on the target area by accident under the influence of gravity alone. The potentials applied to the electrodes of the iris diaphragm are controlled by a control system, which is not part of the invention. The potentials are preferably varied in a determined way during the course of the dose forming process such that the dose obtains the intended properties. While the transfer of particles takes place from the generator through the iris diaphragm to the target area of the substrate member the potential fed to the top electrode of the iris is typically a few hundred volts, positive or negative, in order to attract charged particles. The electrode on the bottom side is typically fed with a potential between zero and some tens of volts in order to slightly repel the charged particles and help guiding particles through the iris diaphragm. The particles emerging from the aperture topside of the iris diaphragm enter the attracting field emanating from the electrode behind the target area of the substrate member. The attracting electrode is typically fed with a potential between 500 and 2000 V. The emerging particles therefore continue on their path in the direction of the target area. During the dose forming process the transfer of particles may be interrupted by the control system, which may create a strong repelling electric field within the iris diaphragm by feeding suitable opposing potentials to the electrodes such that no charged particles can penetrate the aperture of the iris diaphragm.

In order to eliminate the prior art limitations in total dose mass and poor spatial control of the dose layout it is essential to achieve fast and efficient neutralization of charges from the charged powder particles and from the target area of the substrate, i.e. the dose bed, thus eliminating the repelling electric field from the dose during forming. Various methods, e.g. corona, induction or tribo effect may be used to generate equalizing charges, but in a preferred embodiment very quick neutralization is achieved e.g. by arranging a source of positively charged helium ions, so called alpha-particles, near the substrate such that the emitted ions are directed towards the dose and the target area of the substrate. The emitted ions ionize the air and the resulting oxygen and nitrogen ions of both positive and negative charge may be attracted to the dose and the substrate, whereby some of them will hit the dose and the substrate and recombine, neutralizing the accumulated charges in the process. For best results, it is of course necessary to choose an ion source of suitable strength and provide adequate screening to direct the charges towards the dose or, if the target area is larger than the area where the dose is formed, towards the part of the dose where the deposition is taking place.

By immediate, i.e. within a fraction of a second, neutralization of the particle charge once the particle has been deposited on the substrate the negative influence from the particle charge on incoming particles is eliminated. The spatial deposition of the particles is thus vastly improved with no particles settling outside the target area, because the sum of charges on the dose bed and in the dose being formed as a whole is continuously removed in this way eliminating a distorting, repelling electric field from arising. In a typical embodiment of the invention the accumulated charge within the dose and dose bed is regularly removed during the dose forming process as described. If the ion source cannot be positioned and screened to add neutralizing charges directly to the dose and the target area, the dose may be brought within the range of an ion source by a servo device or vice versa, such that the accumulated charge is neutralized at least once and more preferably several times during the forming of the dose. It is also typical that the substrate member must pass by the ion source to neutralize any residual charge from the target area before commencing a dose forming operation.

The electro-powder forms an active dry powder substance or dry powder medication formulation with a fine particle fraction (FPF) presenting of the order 50 % or more of the powder mass with an aerodynamic particle size below 5 µm and provides electrostatic properties with an absolute specific charge per unit mass of the order 0.1 to 25 µC/g after charging, and presents a charge decay rate constant Q₅₀ of more than 0.1 s, a tap density of less than 0.8 g/ml and a water activity a_{w} of less than 0.5.

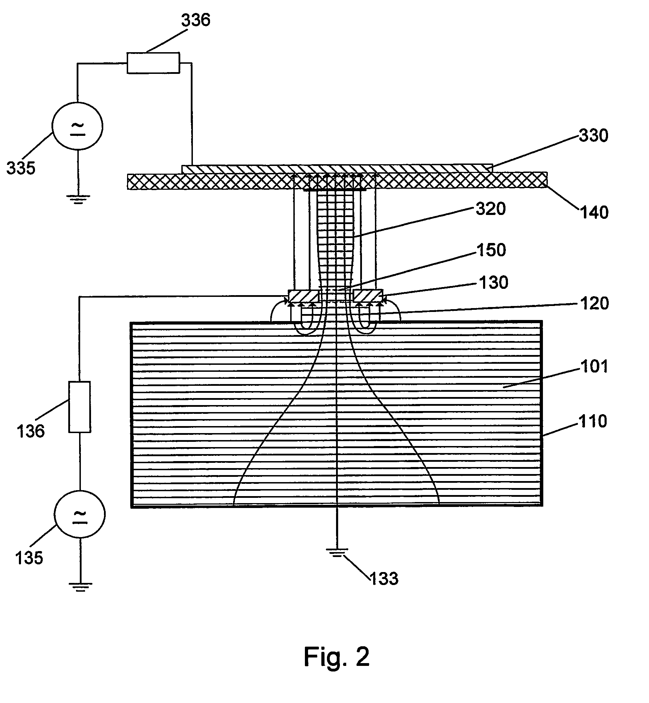

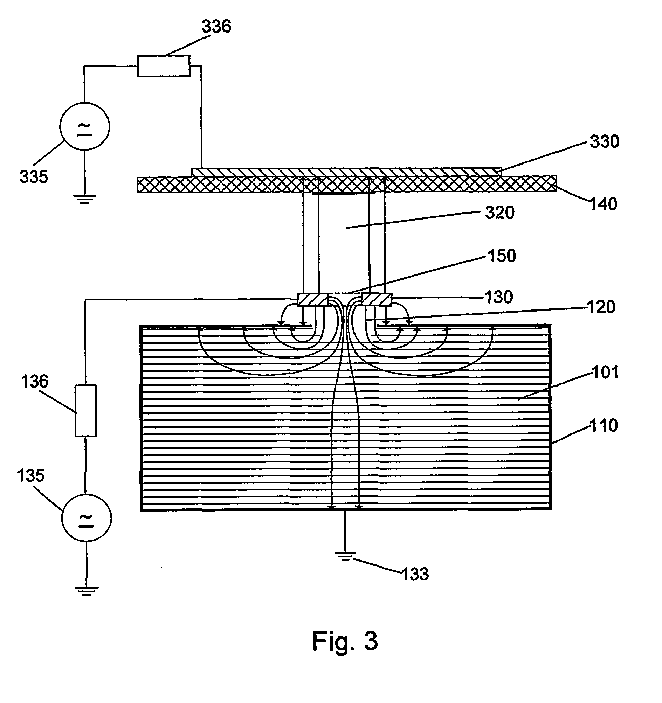

As an illustrative example the dose forming process may best be understood by taking reference in Figure 2. The particle generator **110,** not to be confused with the mentioned ion source, emits particles **101** provided with a positive or negative charge by corona-, tribo- or induction-charging, whereupon the particles enter an imposed first electric field **120**. The type of charge of the particles depends on the powder characteristics, method of charging and materials in the generator so that the majority of the particles are charged either negatively or positively when they are emitted from the generator to take part in the dose forming process. In the following discussion and in the illustrations it is assumed that the emitted particles are positively charged. However, this depends on the properties of the powder and the generator and it is equally possible that the particles are negatively charged, in which case the applied potentials must change signs, but the discussion is still valid. In order to control the dose forming process in terms of total dose mass and dose forming time, the transfer of charged particles from the particle generator to the target area of the substrate member must be controlled. To this end, a first electric field is applied between ground **133** and a first electrode **130** connected to a first voltage source **135,** including source impedance **136.** The electrode is preferably positioned a short distance in the range 0,5 - 25 mm from the substrate member **140** between the particle generator **110** and the substrate member **140.** The strength and direction of the created electric field **120** may be adjusted by adjusting the potential of the electrode within wide limits from a negative to a positive voltage, as set by the voltage source. Charged particles are thereby either attracted to (see Figure 2) or repelled from (see Figure 3) the first electrode, which has at least one aperture **150** of suitable size and shape where charged particles can pass through. Such apertures may be circular, elliptic, square or narrow slits or any other shape in order to suit the dose forming process. In a preferred embodiment, the aperture or apertures are in the range 50 - 5000 µm as main measures. However, particles attracted by the first electrode easily stick to it, which impairs the efficiency of the system and frequent cleaning may become necessary.

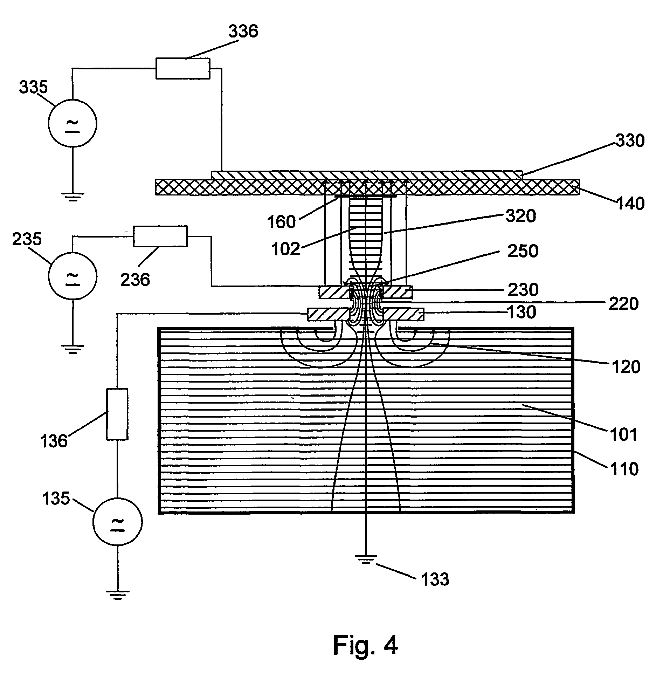

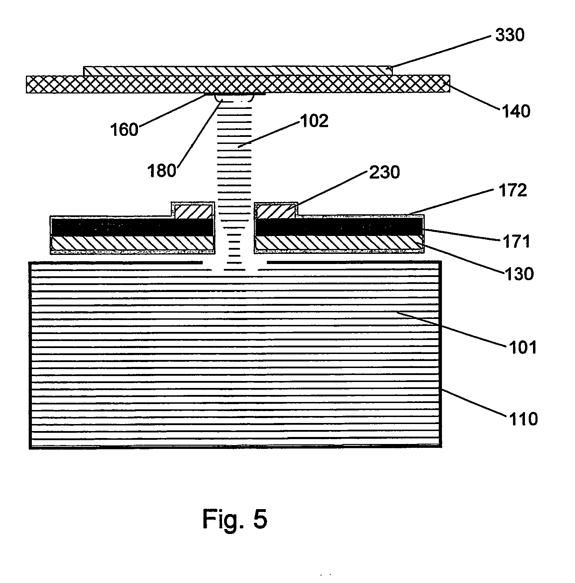

To eliminate the sticking effect and further improve the level of control of the transfer of particles to the target area of the substrate member, an optional second electrode **230** as illustrated in Figure 4, may be introduced. It should be positioned in a plane parallel to the first electrode **130,** in between the first electrode and the substrate at a distance between 0,07 and 2,5 mm from the first electrode. In a preferred embodiment, illustrated in Figure 5, the first and second electrodes are integrated in an isolating wafer member **171** between the electrodes. The outward faces of the electrodes are preferably coated with an isolating coating **172** of a few microns in thickness, e.g. parylene, to stop possible short-circuiting of electrodes by sticking particles. The thickness of the wafer is typically in the range 0,07-2 mm. As an illustrative example the electrodes and the wafer member may be made as a printed circuit board. There are many types commercially available, e.g. in terms of number of possible conductor layers, physical flexibility and thickness.

The wafer member **171** constitutes a physical barrier between the particle generator **110** and the substrate **140** with the dose bed that is the target area **160** for the deposition of charged particles **102.** The distance between the top electrode or electrodes on the top of the wafer member and the substrate is in the range 0,5 to 25 mm. The only possibility for the particles to reach the dose bed is therefore to go through the available apertures of the first and second electrodes and possible extra electrodes, if introduced.

A further third electric field **320** is set up between ground **133** and a third electrode **330** connected to a third voltage source **335.** It is possible to reference the third voltage source to the output of the first or second electrode instead of ground to simplify control of the deposition process. The third electrode is preferably positioned in close proximity behind the substrate member **140** and the dose bed **160,** such that the electric field lines go through the dose bed in the direction of the particle generator **110**. The substrate member may be made of a dielectric or semi-conductive material or even a conducting material or a combination of different such materials. In the case when the material in the dose bed is conductive, the dose bed may constitute the third electrode. The strength and direction of an ensuing third electric field **320** may be adjusted by adjusting the potential of the third electrode within wide limits from a negative to a positive voltage as set by the third voltage source, if connected to the electrode, such that the charged particles are either transported towards or away from the third electrode.

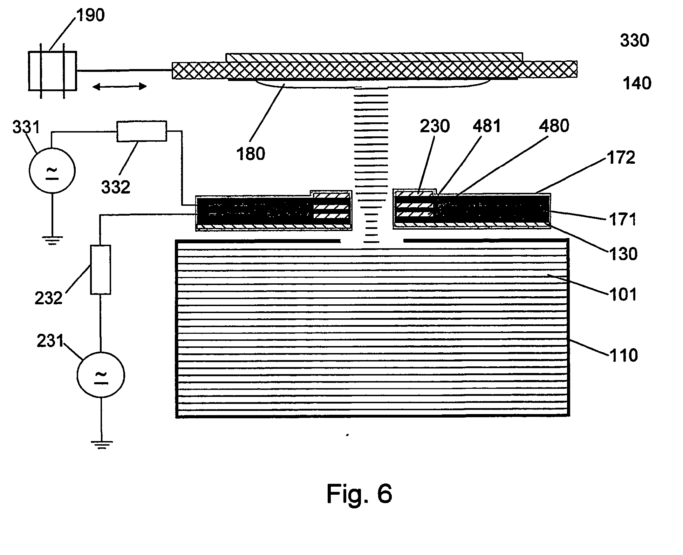

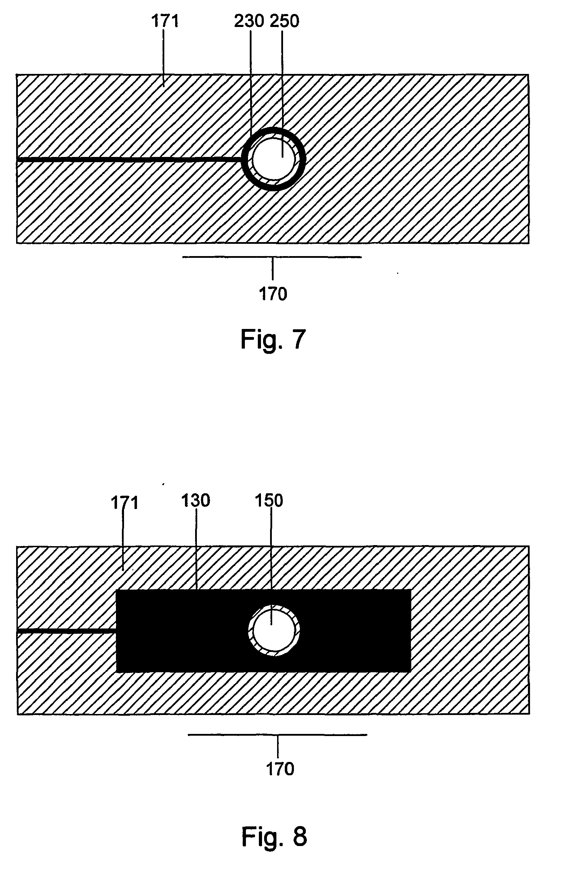

Charged particles **101** emitted from the generator **110** enter the combined electric field resulting from the potentials applied to the first, second and third electrodes respectively. The first electrode alone acts as an electric iris diaphragm device **170** and the addition of the optional second electrode improves the efficiency of the device considerably. A typical embodiment of the electric iris diaphragm is illustrated in Figures 7 and 8, showing the topside and bottom side respectively. The at least one electrode, constituting the iris diaphragm, transfers charged powder particles **101,** emitted from the generator, to the target area **160** on the substrate member in a controlled orderly way in terms of mass, direction and speed, like a printer ink-jet. After passing the iris diaphragm **170,** the particles are accelerated in the third electric field, which may have an AC component, in the direction of the target area of the substrate member, i.e. the dose bed **160,** under the attractive field force caused by the third field emanating from the third electrode behind the dose bed. The bed may be stationary or moving during the distribution of the particles. By utilizing a servomechanism **190,** schematically illustrated in Figure 6, the deposition of the particles can be controlled such that the spatial distribution of the particles on the dose bed area can be controlled arbitrarily.

In order to avoid that particles are deposited at random inside or even outside the target area, because of the local repelling electric field emanating from charges of already deposited particles, the created electrostatic field must be neutralized during the dose forming process. In that case, no significant local repelling electric fields will build up, which may distort the third electric field and weaken its attractive power, leading to a scattering of incoming charged particles. If charges accumulating in the dose and dose bed are frequently neutralized new particles will automatically go from the output of the iris diaphragm to the closest point of the dose bed such that there is a sharp distinction between the formed dose and the surrounding areas of the substrate.

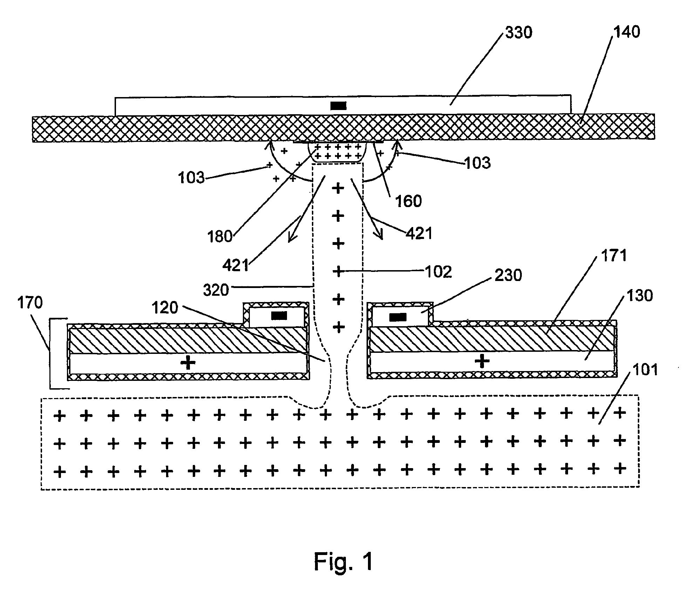

The problem presented by the created repelling field caused by the accumulated charged particles is illustrated in Figure 1. Basically charged powder particles **101** (positive or negative, positive charge is assumed in Figure 1) are transported by an electric field **120** through an iris diaphragm **170** and then by a field **320** towards the target area **160** of the substrate member **140,** where the particles **102** are deposited forming the dose **180.** The more particles that are deposited the less free area is available for new particles. The accumulated charged particles create a local electric field, which exerts a force **421,** which in turn repels newcomers **102,** forcing some **103** to settle outside the target area or to be retained and wasted on the walls of the dose forming apparatus. In a preferred embodiment, electro-powder is used, but other medication powders may be possible to use, which is easily recognized by people of ordinary skill in the art.

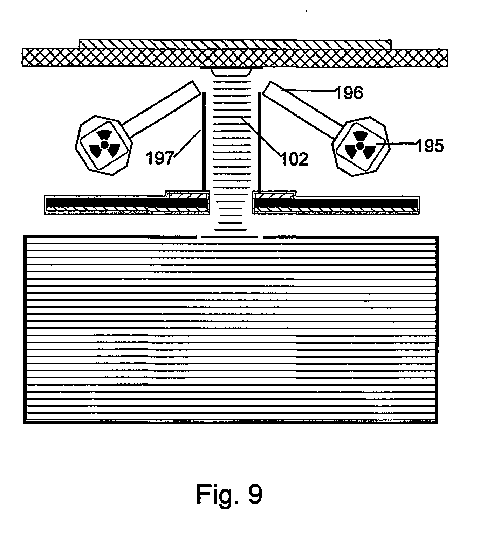

A key element of the invention is schematically illustrated in Figures 9, 10 and 11 i.e. the element neutralizing the accumulated charge of particles deposited on the dose bed. Various methods, e.g. corona, induction or tribo effect may be used to generate neutralizing charges, but in a preferred embodiment, a radioactive source **195** of alpha-particles (positively charged helium atoms) has been found to be most efficient. These sources are readily commercially available, e.g. from NRD LLC, Grand Island, N.Y. and are specifically used to discharge electrically charged objects. The alpha particles are scattered uniformly in all directions from, for instance, a point source and ionize the surrounding air creating both positive and negative ions. The new ions are attracted to oppositely charged particles and other charged objects in the vicinity and recombine to form regular atoms using the surplus charge of the objects with which they collide. The active range from the ion source is only a few centimeters. It is very easy to stop the alpha particles within the active range by putting any solid material in the way, like a sheet of paper. A preferred radioactive point source is model P-2042 Nuclespot^{™}, which is based on Polonium-210, but other models are available to suit all kinds of applications. Polonium-210 is currently used and has a long record of use in all kinds of industry where static electricity is a problem. The radiation does not affect the medication powder in any way besides neutralizing the charge and it leaves no residue besides helium atoms (inert gas), which are the result of the alpha particles colliding with air molecules taking up two electrons from oxygen or nitrogen atoms. In their effort to recombine, a current of ions is established that quickly neutralizes charged objects and surfaces within the active range of the radioactive point source.

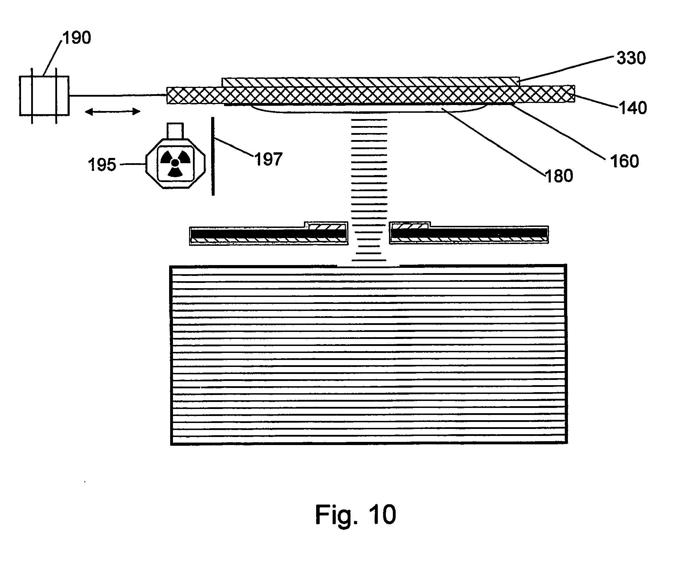

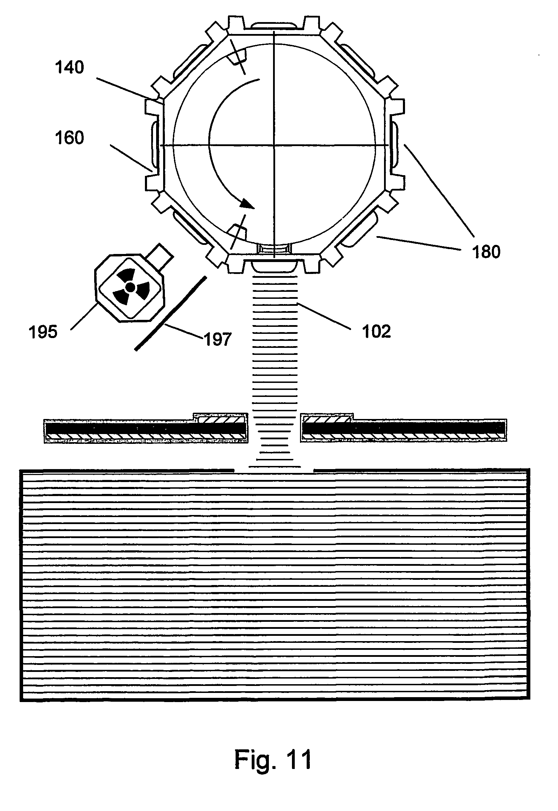

In one embodiment, illustrated in Figure 9, it is possible to direct the alpha particles by designing at least one direction member **196** pointing to the spot on the dose bed where the powder particles **102** are deposited, such that immediately after the deposition the charge of the individual particles is neutralized. In a different embodiment, the ion source **195** is put outside the spot where the dose is formed, illustrated in Figure 10. The previously mentioned servo device **190** is now set up to withdraw the substrate **140** with the dose bed **160** after only a partial dose forming operation before too many charged particles **102** have been deposited in order to neutralize the accumulated charges from the dose bed and the dose **180** by exposing the dose and substrate to the emitted ions from the source. Yet another embodiment is illustrated in Figure 11, showing a typical arrangement where the substrate member is a cassette **140** carrying at least one target area **160** for dose forming and an ion source directed towards the target area, which will receive the next dosing in a repeated sequence of dose forming operations. Generally, for all embodiments it is necessary to include screens **197,** which will absorb charges that otherwise risk interfering with charged particles being transported in the electric fields set up to control the transport, distribution and final deposition of the particles in the dose forming process.

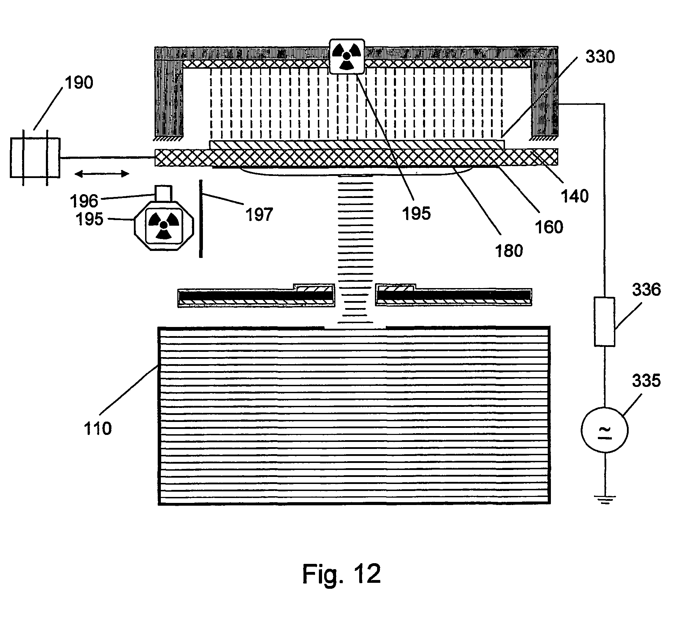

In a different embodiment physical constraints may exist in a member carrying one or more substrate members intended for doses, which make it difficult or impossible to arrange a contacting of an electrode behind the substrate member necessary for creating the third electric field as previously discussed. In such case, illustrated schematically in Figure 12, a separate ion source **195** may advantageously be applied to make electrical contact with the third electrode **330** behind the substrate member **140** without actual physical contact. The emitted alpha particles ionize the air, which acts as an electric conductor between the ion source and the third electrode, which must be electrically conductive. The ion source should be of suitable size and placed within its working range 0-30 mm from the third electrode on the backside of the substrate member. If the metal shell of the ion source is connected to the third voltage source **335** with effective internal impedance **336,** which now includes the impedance of the air gap, part of the applied voltage will also be present as a potential on the third electrode, such that the third field can be fully controlled.

It is worth noting that for all practical embodiments of the invention depositing large amounts of powder is no problem, provided the negative influence of the created electrostatic field from the accumulated charged particles constituting the dose and of the stray charges of the substrate is neutralized by removing the charges as described in the foregoing description. Then, the field strength from the third electrode is approximately constant through the substrate and developing dose. The distribution process and the forming of the dose are not sensitive to variations between particles in total charge or specific charge. As long as a particle has a charge of the right type and manages to pass the screening in the iris diaphragm, it will automatically be deposited onto the dose bed as long as the field exists. Provided suitable measuring instruments are put to use for monitoring the dose while it is formed, it is easy to control the described dose forming process on-line, using standard prediction, feed-forward and feed-back control methods, in combination if necessary.

What has been said in the foregoing is by example only and many variations of the disclosed embodiments may be obvious to a person of ordinary skill in the art, without departing from the scope of the invention as defined by the appended claims.

## Claims

1. A method for quick neutralization of a created electrostatic field formed by a multitude of basically charged particles constituting a medication powder deposited onto a defined target area of a substrate member in a dose forming process, **characterized by** the steps of
applying a device comprising a source (195) of electric charges emitting positive or negative or both types of charges within range of a dose (180) being formed onto the defined target area of the substrate member (140);
arranging a transmission of equalizing electric charges to a multitude of charged particles, deposited onto the target area (160) of the substrate member, for the purpose of equalizing the charge in the dose being formed and also equalizing stray charges on the substrate surface.

2. The method according to claim 1, **characterized by** the steps of
controlling an electrode potential without physically contacting the electrode
establishing electric contact over an air gap between an electrode (330) behind a substrate member (140) and a voltage source (335) by
using a source (195) of electric charges for emitting sufficient charges into an air gap to establish an electric contact between an electrode (330) and a voltage source (335) resulting in a source impedance (336) less than 1 GΩ;
connecting a controlled potential from the voltage source through the air gap to the electrode (330) thus creating a necessary electric field emanating from the electrode for transportation of the charged particles (102) to the target area (160) in the dose forming process.

3. The method according to claim 1 or 2, **characterized by** arranging an ion source (195) as the source of electric neutralizing charges, and optionally providing the ion source with direction members (196) and screens (197).

4. The method according to claim 1 or 2, **characterized by** arranging a corona-type charge generator (195) as the source of electric neutralizing charges, and optionally providing the source (195) with direction members (196) and screens (197).

5. The method according to claim 1 or 2, **characterized by** arranging an induction-type charge generator (195) as the source of electric neutralizing charges, and optionally providing the source (195) with direction members (196) and screens (197).

6. The method according to claim 1 or 2, **characterized by** arranging a tribo-type charge generator (195) as the source of electric neutralizing charges, and optionally providing the source with direction members (196) and screens (197).

7. The method according to claim 1 or 2, **characterized by** arranging a continuous transmission of neutralizing charges from a source of charges (195) directed towards a multitude of charged particles (102) deposited on the target area (160) of the substrate member (140) during a dose forming process.

8. The method according to claim 1 or 2, **characterized by** positioning the source (195) of neutralizing charges out of range of a dose forming area such that the substrate member (140) with the defined target area (160) and the dose (180) being formed can be brought within range of emitted charges by means of a servo device (190), such that the dose (180) being formed is temporarily brought in a position to get the accumulated local electrostatic field neutralized at least once during the forming of the dose.

9. A device for quick neutralization of a created electrostatic field formed by a multitude of basically charged particles (102) constituting a medication powder deposited on a defined target area (160) of a substrate member (140) in a dose forming process, **characterized by** having
a source (195) of electric neutralizing charges emitting positive or negative or both types of charges within a working range of a medication dose (180) being formed on the target area (160) of the substrate member (140);
a mechanical servo device (190) for transmitting the charges from the source (195) of electric charges into the multitude of charged particles (102) deposited onto the target area (160) of the substrate member (140) for a purpose of neutralizing the charge of the dose (180) in forming and stray charges on the substrate (140) surface.

10. The device according to claim 9, **characterized by**
an electrode potential which is controlled with no physical contact to the electrode,
a mechanical servo device (190) for transmitting the charges from the source (195) of electric charges into the multitude of charges particles (102) deposited onto the target area (160) of the substrate member (140) for a purpose of neutralizing the charge of the dose (180) in forming and stray charges on the face of the substrate (140);
a source (195) of electric charges emitting sufficient charges into an air gap to establish an electric contact between an electrode (330) and a voltage source (335) resulting in an impedance less than 1 GΩ;
a controlled potential connected from the voltage source (335) through the air gap to the electrode (330) thus creating a necessary electric field emanating from the electrode for transportation of the charged particles (103) to the defined target area (160) of the dose forming process.

11. The device according to claim 9 or 10, **characterized in that** the voltage source (335) has an impedance less than 100 MΩ.

12. The device according to claim 9 or 10, **characterized in that** the source of electric neutralizing charges is an ion source (195) which is optionally provided with direction members (196) and screens (197).

13. The device according to claim 9 or 10, **characterized in that** the source of electric neutralizing charges (195) is a corona-type charge generator, which is optionally provided with direction members (196) and screens (197).

14. The device according to claim 9 or 10, **characterized in that** the source of electric neutralizing charges (195) is an induction-type charge generator, which is optionally provided with direction members (196) and screens (197).

15. The device according to claim 9 or 10, **characterized in that** the source of electric neutralizing charges (195) is a tribo-type charge generator, which is optionally provided with direction members (196) and screens (197).

16. The device according to claim 9 or 10, **characterized in that** the source of neutralizing charges (195) continuously transmits neutralizing charges directed towards the multitude of charged particles (102) deposited on the target area (160) of the substrate member (140).

17. The device according to claim 9, **characterized in that** the source of neutralizing charges is located out of range of the dose forming area but in a position where the substrate member (140) and the dose being formed can be brought within range of the emitted neutralizing charges by means of a servo device (190), such that the dose (180) being formed is temporarily brought in position to get the accumulated local electrostatic field neutralized at least once during forming of the dose.

## Patentansprüche

1. Verfahren zur schnellen Neutralisierung eines erzeugten elektrostatischen Feldes, gebildet durch eine Vielzahl von im Wesentlichen geladenen Teilchen, welche ein Medikationspulver darstellen, das auf einem definierten Zielbereich eines Substratelements in einem Dosis bildenden Vorgang abgelegt ist, **gekennzeichnet durch** die Schritte Anlegen einer Vorrichtung mit einer Quelle (195) elektrischer Ladungen, welche positive oder negative oder beide Arten von Ladungen innerhalb eines Bereichs einer Dosis (180), die auf dem definierten Zielbereich des Substratelements (140) ausgebildet ist, ausgibt; Anordnen einer Übertragung von ausgleichenden elektrischen Ladungen zu einer Vielzahl von geladenen Teilchen, die auf dem Zielbereich (160) des Substratelements abgelegt sind, zum Zwecke des Ausgleichs der Ladung in zu bildenden Dosis und außerdem des Ausgleichs von Streuladungen auf der Substratoberfläche.

2. Verfahren nach Anspruch 1, **gekennzeichnet durch** die Schritte
Steuern eines Elektrodenpotenzials ohne physikalisches Berühren der Elektrode,
Einrichten eines elektrischen Kontakts über einen Luftspalt zwischen einer Elektrode (330) hinter einem Substratelement (140) und einer Spannungsquelle (335) **durch** Verwenden einer Quelle (195) elektrischer Ladungen zum Ausgeben ausreichender Ladungen in einen Luftspalt, um einen elektrischen Kontakt zwischen einer Elektrode (330) und einer Spannungsquelle (335) herzustellen, was zu einer Quellenimpedanz (336) von weniger als 1 GΩ führt;
Verbinden eines gesteuerten Potenzials von der Spannungsquelle **durch** den Luftspalt zu der Elektrode (330), wodurch ein notwendiges elektrisches Feld erzeugt wird, das von der Elektrode zum Transport der geladenen Teilchen (102) zu dem Zielbereich (160) in dem Dosis bildenden Vorgang ausstrahlt.

3. Verfahren nach Anspruch 1 oder 2, **gekennzeichnet durch** Anordnen einer Ionenquelle (195) als die Quelle der elektrisch neutralisierenden Ladungen und optional Ausstatten der Ionenquelle mit Richtungselementen (196) und Blenden (197).

4. Verfahren nach Anspruch 1 oder 2, **gekennzeichnet durch** Anordnen eines Korona-Ladungsgenerators (195) als die Quelle der elektrisch neutralisierenden Ladungen und optional Ausstatten der Quelle (195) mit Richtungselementen (196) und Blenden (197).

5. Verfahren nach Anspruch 1 oder 2, **gekennzeichnet durch** Anordnen eines Induktions-Ladungsgenerators (195) als die Quelle der elektrisch neutralisierenden Ladungen und optional Ausstatten der Quelle (195) mit Richtungselementen (196) und Blenden (197).

6. Verfahren nach Anspruch 1 oder 2, **gekennzeichnet durch** Anordnen eines Tribo-Ladungsgenerators (195) als die Quelle der elektrisch neutralisierenden Ladungen und optional Ausstatten der Quelle (195) mit Richtungselementen (196) und Blenden (197).

7. Verfahren nach Anspruch 1 oder 2, **gekennzeichnet durch** Anordnen einer ständigen Übertragung von neutralisierenden Ladungen von einer Quelle der Ladungen (195), gerichtet in Richtung zu einer Vielzahl von geladenen Teilchen (102), die auf dem Zielbereich (160) des Substratelements (140) während eines Dosis bildenden Vorgangs abgelegt sind.

8. Verfahren nach Anspruch 1 oder 2, **gekennzeichnet durch** Positionieren der Quelle (195) der neutralisierenden Ladungen außerhalb des Bereichs eines Dosis bildenden Bereichs, so dass das Substratelement (140) mit dem definierten Zielbereich (160) und die zu bildende Dosis (180) mittels einer Servovorrichtung (190) innerhalb eines Bereichs der ausgegebenen Ladungen gebracht werden kann, so dass die zu bildende Dosis (180) vorübergehend in eine Position gebracht wird, damit das angesammelte lokale elektrostatische Feld wenigstens einmal während der Bildung der Dosis neutralisiert wird.

9. Vorrichtung zur schnellen Neutralisierung eines erzeugten elektrostatischen Feldes, gebildet durch eine Vielzahl von hauptsächlich geladenen Teilchen (102), welche ein Medikationspulver darstellen, das auf einem definierten Zielbereich (160) eines Substratelements (140) in einem Dosis bildenden Vorgang abgelegt ist, **gekennzeichnet durch** eine Quelle (195) elektrisch neutralisierender Ladungen, welche positive oder negative oder beide Arten von Ladungen innerhalb eines Arbeitsbereichs einer Medikationsdosis (180), die auf dem Zielbereich (160) des Substratelements (140) ausgebildet wird, ausgibt;
eine mechanische Servovorrichtung (190) zum Übertragen der Ladungen von der Quelle (195) elektrischer Ladungen in die Vielzahl geladener Teilchen (102), die auf dem Zielbereich (160) des Substratelements (140) abgelegt sind, zum Zwecke der Neutralisierung der Ladung der Dosis (180) beim Ausbilden und von Streuladungen auf der Oberfläche des Substrats (140).

10. Vorrichtung nach Anspruch 9, **gekennzeichnet durch**
ein Elektrodenpotenzial, das ohne physikalischen Kontakt zur Elektrode gesteuert wird,
eine mechanische Servovorrichtung (190) zum Übertragen der Ladungen von der Quelle (195) elektrischer Ladungen in die Vielzahl geladener Teilchen (102), die auf dem Zielbereich (160) des Substratelements (140) abgelegt sind, zum Zwecke der Neutralisierung der Ladung der Dosis (180) beim Ausbilden und von Streuladungen auf der Oberfläche des Substrats (140);
eine Quelle (195) elektrischer Ladungen, welche ausreichende Ladungen in einen Luftspalt ausgibt, um einen elektrischen Kontakt zwischen einer Elektrode (330) und einer Spannungsquelle (335) herzustellen, was zu einer Impedanz von weniger als 1 GΩ führt;
ein gesteuertes Potenzial, verbunden von der Spannungsquelle (335) **durch** den Luftspalt zu der Elektrode (330), wodurch ein notwendiges elektrisches Feld erzeugt wird, das von der Elektrode zum Transport der geladenen Teilchen (103) zu dem definierten Zielbereich (160) in dem Dosis bildenden Vorgang ausstrahlt.

11. Vorrichtung nach Anspruch 9 oder 10, **dadurch gekennzeichnet, dass** die Spannungsquelle (335) eine Impedanz von weniger als 100 MΩ aufweist.

12. Vorrichtung nach Anspruch 9 oder 10, **dadurch gekennzeichnet, dass** die Quelle der elektrisch neutralisierenden Ladungen eine Ionenquelle (195) ist, die optional mit Richtungselementen (196) und Blenden (197) ausgestattet ist.

13. Vorrichtung nach Anspruch 9 oder 10, **dadurch gekennzeichnet, dass** die Quelle der elektrisch neutralisierenden Ladungen (195) ein Korona-Ladungsgenerator ist, der optional mit Richtungselementen (196) und Blenden (197) ausgestattet ist.

14. Vorrichtung nach Anspruch 9 oder 10, **dadurch gekennzeichnet, dass** die Quelle der elektrisch neutralisierenden Ladungen (195) ein Induktions-Ladungsgenerator ist, der optional mit Richtungselementen (196) und Blenden (197) ausgestattet ist.

15. Vorrichtung nach Anspruch 9 oder 10, **dadurch gekennzeichnet, dass** die Quelle der elektrisch neutralisierenden Ladungen (195) ein Tribo-Ladungsgenerator ist, der optional mit Richtungselementen (196) und Blenden (197) ausgestattet ist.

16. Vorrichtung nach Anspruch 9 oder 10, **dadurch gekennzeichnet, dass** die Quelle der neutralisierenden Ladungen (195) ständig neutralisierende Ladungen überträgt, gerichtet in Richtung zur Vielzahl von geladenen Teilchen (102), die auf dem Zielbereich (160) des Substratelements (140) abgelegt sind.

17. Vorrichtung nach Anspruch 9, **dadurch gekennzeichnet, dass** die Quelle der neutralisierenden Ladungen außerhalb des Bereichs des Dosis bildenden Bereichs liegt, aber an einer Position, an der das Substratelement (140) und die zu bildende Dosis mittels einer Servovorrichtung (190) innerhalb eines Bereichs der ausgegebenen neutralisierenden Ladungen gebracht werden können, so dass die zu bildende Dosis (180) vorübergehend in eine Position gebracht wird, damit das angesammelte lokale elektrostatische Feld wenigstens einmal während des Ausbildens der Dosis neutralisiert wird.

## Revendications

1. Procédé de neutralisation rapide d'un champ électrostatique créé par une multitude de particules basiquement chargées constituant une poudre médicamenteuse déposée sur une zone cible définie d'un élément de substrat au cours d'une opération de formation de dose, **caractérisé par** les étapes suivantes :
mettre en oeuvre un dispositif comprenant une source (195) de charges électriques émettant des charges positives, négatives, ou des deux types, dans les limites d'une dose (180) en cours de formation sur la zone cible définie de l'élément de substrat (140) ;
assurer un transfert de charges électriques d'égalisation à une multitude de particules chargées, déposées sur la zone cible (160) de l'élément de substrat, aux fins d'égaliser la charge dans la dose en cours de formation et également d'égaliser des charges parasites sur la surface du substrat.

2. Procédé selon la revendication 1, **caractérisé par** les étapes suivantes :
régler un potentiel d'électrode sans établir de contact physique avec l'électrode ;
établir un contact électrique à travers un intervalle d'air entre une électrode (330) située derrière un élément de substrat (140) et une source de potentiel (335) en utilisant une source (195) de charges électriques pour émettre des charges suffisantes dans un intervalle d'air de manière à établir un contact électrique entre une électrode (330) et une source de tension (335) conduisant à une impédance de source (336) inférieure à 1 GΩ ;
relier un potentiel réglé de la source de tension à travers l'intervalle d'air à une électrode (330), créant ainsi un champ électrique nécessaire émis par l'électrode de manière à transporter les particules chargées (102) jusqu'à la région cible (160) pendant l'opération de formation de dose.

3. Procédé selon la revendication 1 ou 2, **caractérisé en ce qu'**il prévoit la mise en oeuvre d'une source d'ions (195) comme source de charges de neutralisation électrique, et prévoit, en option, de doter la source d'ions d'éléments d'orientation (196) et d'écrans (197).

4. Procédé selon la revendication 1 ou 2, **caractérisé en ce qu'**il prévoit la mise en oeuvre d'un générateur de charges du type à effet couronne (195) comme source de charges de neutralisation électrique, et prévoit, en option, de doter la source (195) d'éléments d'orientation (196) et d'écrans (197).

5. Procédé selon la revendication 1 ou 2, **caractérisé en ce qu'**il prévoit la mise en oeuvre d'un générateur de charges du type à induction (195) comme source de charges de neutralisation électrique, et prévoit, en option, de doter la source (195) d'éléments d'orientation (196) et d'écrans (197).

6. Procédé selon la revendication 1 ou 2, **caractérisé en ce qu'**il prévoit la mise en oeuvre d'un générateur de charges du type triboélectrique (195) comme source de charges de neutralisation électrique, et prévoit, en option, de doter la source (195) d'éléments d'orientation (196) et d'écrans (197).

7. Procédé selon la revendication 1 ou 2, **caractérisé en ce qu'**il prévoit un transfert continu de charges de neutralisation provenant d'une source de charges (195) dirigées vers une multitude de particules chargées (102) déposées sur la zone cible (160) de l'élément de substrat (140) pendant une opération de formation de dose.

8. Procédé selon la revendication 1 ou 2, **caractérisé en ce qu'**il prévoit le positionnement de la source (195) de charges de neutralisation hors des limites d'une zone de formation de dose, de telle manière que l'élément de substrat (140) comportant la zone cible définie (160) et la dose (180) en cours de formation peut être amené dans les limites des charges émises au moyen d'un dispositif asservi (190), de sorte que la dose (180) en cours de formation soit amenée temporairement dans une position telle que le champ électrostatique local cumulé soit neutralisé au moins une fois pendant l'opération de formation de dose.

9. Dispositif de neutralisation rapide d'un champ électrostatique créé par une multitude de particules basiquement chargées (102) constituant une poudre médicamenteuse déposée sur une zone cible définie (160) d'un élément de substrat (140) au cours d'une opération de formation de dose, **caractérisé en ce qu'**il comprend :
une source (195) de charges électriques de neutralisation émettant des charges positives, négatives, ou des deux types, dans les limites d'une région active d'une dose médicamenteuse (180) en cours de formation sur la zone cible (160) de l'élément de substrat (140) ;
un dispositif asservi mécanique (190) pour transmettre les charges depuis la source (195) de charges électriques vers la multitude de particules chargées (102) déposées sur la zone cible (160) de l'élément de substrat (140) aux fins de neutraliser la charge de la dose en formation (180) et des charges parasites sur la surface de substrat (140).

10. Dispositif selon la revendication 9, **caractérisé en ce qu'**il comprend :
un potentiel d'électrode qui est réglé sans établir de contact physique avec l'électrode ;
un dispositif asservi mécanique (190) pour transmettre les charges depuis la source (195) de charges électriques vers la multitude de particules chargées (102) déposées sur la zone cible (160) de l'élément de substrat (140) aux fins de neutraliser la charge de la dose (180) en formation et des charges parasites sur la surface de substrat (140) ;
une source (195) de charges électriques émettant des charges suffisantes dans un intervalle d'air de manière à établir un contact électrique entre une électrode (330) et une source de tension (335) conduisant à une impédance inférieure à 1 GΩ ;
un potentiel soumis à réglage relié entre la source de tension (335) à travers l'intervalle d'air et l'électrode (330), créant ainsi un champ électrique nécessaire émis par l'électrode de manière à transporter les particules chargées (102) vers la région cible définie (160) dans l'opération de formation de dose.

11. Dispositif selon la revendication 9 ou 10, **caractérisé en ce que** la source de tension (335) présente une impédance inférieure à 100 MΩ.

12. Dispositif selon la revendication 9 ou 10, **caractérisé en ce que** la source de charges électriques de neutralisation est une source d'ions (195) comportant, en option, des éléments de pointage (196) et des écrans (197).

13. Dispositif selon la revendication 9 ou 10, **caractérisé en ce que** la source de charges électriques de neutralisation (195) est un générateur du type à effet couronne comportant, en option, des éléments de pointage (196) et des écrans (197).

14. Dispositif selon la revendication 9 ou 10, **caractérisé en ce que** la source de charges électriques de neutralisation (195) est un générateur de charges du type à induction comportant, en option, des éléments de pointage (196) et des écrans (197).

15. Dispositif selon la revendication 9 ou 10, **caractérisé en ce que** la source de charges électriques de neutralisation (195) est un générateur de charges du type triboélectrique comportant, en option, des éléments de pointage (196) et des écrans (197).

16. Dispositif selon la revendication 9 ou 10, **caractérisé en ce que** la source de charges de neutralisation (195) transmet en continu des charges de neutralisation dirigées vers la multitude de particules chargées (102) déposées sur la zone cible (160) de l'élément de substrat (140).

17. Dispositif selon la revendication 9, **caractérisé en ce que** la source de charges de neutralisation est située hors des limites de la zone de formation de dose mais à un emplacement où l'élément de substrat (140) et la dose en cours de formation peuvent être amenés dans la zone atteinte par les charges de neutralisation émises au moyen d'un dispositif asservi (190), de telle manière que la dose (180) en cours de formation soit amenée temporairement dans une position telle que le champ électrostatique local cumulé soit neutralisé au moins une fois pendant l'opération de formation de dose.
